# EUROPEAN PATENT APPLICATION

(11) **EP 1 394 251 A1**
(43) Date of publication of application: **03.03.2004**
(21) Application number: 02018855.3
(22) Date of filing: 23.08.2002
(51) Int. Cl.: C12N 15/10

(54) **Method for the selective randomization of polynucleotides**

(71) Applicant: Direvo Biotech AG, 50829 Köln (DE)
(72) Inventor: Koltermann, Andre, Direvo BioTech AG, 50829 Köln (DE); Kettling, Ulrich, Direvo BioTech AG, 50829 Köln (DE); Pilling, Jens, Direvo BioTech AG, 50829 Köln (DE); Spangenberg, Oliver, Direvo BioTech AG, 50829 Köln (DE)
(74) Representative: Helbing, Jörg, Dr. Dipl.-Chem.

(57) **Abstract**

The present invention provides a method for the randomization of polynucleotides at specific sites which comprises providing a double stranded polynucleotide sequence having at least one differing site and selectively randomizing the polynucleotide at or in the proximity to the differing sites.

## Description

The present invention provides a method for the randomization of polynucleotides at specific sites which comprises providing a double stranded polynucleotide sequence having at least one differing site and selectively randomizing the polynucleotide at or in the proximity to the differing sites.

### Background of the Invention

The basic concept of genetic engineering is the identification of a gene of interest in nature, followed by the transfer of this gene to a production organism and the production of the corresponding gene product - be it an enzyme, an antibody or a secondary metabolite - by fermentation. Heterologous gene expression has been an epoche-making step for a simple reason - gene products of enormous value became available in quantities that were far from reach by extraction from natural sources. However, nature certainly did not evolve molecules to serve as a biopharmaceutical, as an industrial enzyme or as a biocatalyst for chemical processes. Therefore, it became very early obvious that the quantitative improvement could be multiplied by a qualitative improvement. Qualitative improvement means modifying the properties or the composition of one or several gene products of interest, with the aim to improve their technical or medical applicability. If the gene products are proteins, e.g. enzymes or antibodies, this qualitative improvement has been termed protein engineering. Other applications have been termed analogously. For example, when dealing with metabolites, the process has been called metabolic engineering. The improvement of bacterial strains has been called strain engineering, etc. Today, there is an increasing demand for such engineering technologies, allowing to engineer gene products to become new functional ingredients in nutrients or consumer products, new catalysts for the chemical industry, or new drugs to target diseases that are not or not sufficiently treatable yet.

Independently of the nature of the gene product of interest, engineering to improve the quality of this gene product relies on the modification of the gene sequence or polynucleotide that encodes it. A wide variety of techniques for the modification of gene sequences are known. In general, one has to distinguish between methods for the generation of new combinations of existing sequence parts on the one hand and methods for the generation of new sequences by mutagenesis on the other hand. Both classes of techniques can further be classified into deterministic and random techniques. While deterministic methods have the aim to generate one or a few polynucleotides with specific sequences, random techniques, on the other hand, have the aim to generate polynucleotides with at least partially random sequences. See Table 1 for a general overview on techniques for the modification of gene sequences.

**Table 1:**

| Techniques for the modification of gene sequences | | |
|---|---|---|
| | Deterministic: | Random: |
| Generation of new combinations of sequences: | Insertion or joining to-gether of specific sequences (more generally known as recombinant DNA technology) | Random recombination - homologous or heterologous - of sequence parts (DNA shuffling, RCR, Step, Itchy) |
| Generation of new sequences by mutagenesis: | Defined exchange of one or more nucleotides (known as site-specific mutagenesis, e.g. Kunkel method, etc.) | Random mutagenesis (mutagenic PCR, cassette mutagenesis, method of the invention) |

Techniques for the deterministic generation of new combinations of sequence parts insert a specific sequence into another sequence at a specific site or, more generally, join two or more specific sequences in a specific order together. Insertion or joining is traditionally done by cutting sequences at specific sites with restriction enzymes and ligating the resulting pieces together by means of ligase enzymes. Alternatively, recently developed techniques use recombinase enzymes for the same purpose. These techniques are generally known as recombinant DNA technology. Random recombination techniques, on the other hand, combine sequence parts at more or less randomly chosen positions, i.e. generate in principle all possible combinations of sequences that are provided. This can either be done homologously, i.e. by joining analogous sequence parts from different source sequences, or heterologously, i.e. by joining non-analogous sequence parts from different source sequences.

Techniques for the deterministic generation of new sequences change one or more nucleotides at specific sites of a polynucleotide for a different nucleotide. Although being specific with regard to the resulting polynucleotide sequence - and not only with regard to the site of the exchange - these methods are traditionally called site-specific mutagenesis methods. A well-known technique enabling the defined exchange of a specific nucleotide to be chosen in a polynucleotide is the protocol according to Kunkel (PNAS, 82(2):488, 1985).Techniques for the random generation of new sequences, on the other hand, lead to pools of polynucleotides with sequences that are not determined. With regard to the position, this randomization of nucleotides can either be done again randomly over the whole gene sequence, e.g. by modified PCR protocols, or at defined positions or regions, e.g. by exchanging sequence parts with their randomized counterparts.

In general, deterministic techniques have the aim to generate one or a few desired sequences. These gene sequences are either known or expected to lead to improved gene products. Accordingly, deterministic techniques rely either on the knowledge or on the theoretical modeling of the relation between genotypes and phenotypes of gene sequences. Random techniques do not require knowledge of the relation between genotypes and phenotypes of gene sequences, but instead rely on methods for the efficient identification of gene sequences with a desired phenotype out of the pool of random sequences that are generated.

There exists a simple relation between the degree of modification of a gene sequence and the intended improvement factor of the gene product: the higher the intended improvement factor is, the more modifications of the gene sequence are required. Random recombination techniques are limited in this respect, since these techniques do not generate new sequences but only recombine existing ones. Techniques for the random generation of new sequences, i.e. random mutagenesis techniques, are therefore of enormous importance, since only these techniques allow the introduction of new variety and thereby the generation of new sequences that are not existent in nature yet.

Random mutagenesis techniques either introduce random mutations homogeneously over the entire target sequence, or enable the localization of the randomization to discrete positions or regions of the polynucleotide of interest.

Most methods for homogeneous randomization of entire target sequences work by increasing the frequency of misincorporations during polynucleotide amplification.

Lehtovaara and coworkers (Lehtovaara, P.M. et al., Protein Eng. 2 (1): 63,1988) describe a method for introducing all types of base substitution mutations randomly into a nucleic acid. The method comprises the extension of a primer hybridized to the nucleic acid to be mutagenized in four separate reactions - one for each nucleotide - to generate a population of molecules, each copied from the template and terminating at all possible positions of the particular nucleotide; misincorporation of nucleotides at the variable 3' ends generated before; and completion of the molecules to forms that can be amplified and cloned.

Cadwell and Joyce (PCR Methods Appl. 2 (1): 28, 1992; PCR Methods Appl. 3 (6): 136, 1994) describe a random mutagenesis technique referred to as mutagenic PCR. The modified polymerase chain reaction is performed under conditions that reduce the fidelity of nucleotide incorporation during DNA synthesis by using unequal concentrations of the four dNTPs and adding manganese instead of magnesium ions.

Virnekas et al. (Nucleic Acids Res. 22 (25): 5600,1994) describe a random mutagenesis technique that uses trinucleotide phosphoramidites. These trinucleotide represent codons for all 20 amino acids, and are used as reagents for the chemical synthesis of mutagenized oligonucleotides.

Besides these techniques for homogeneous random mutagenesis of nucleic acids, there are several methods published for the selective randomization of specific sites of a polynucleotide sequence.

Wells et al. (Gene 34(2-3): 315, 1985) describe a method for the randomization of a sequence of interest at specific sites or regions. The method uses mutagenic oligodeoxynucleotide cassettes to generate random nucleotide substitutions. The introduction of a DNA cassette allows saturation of a target amino acid codon with multiple mutations. This procedure of complete randomization of the amino-acid sequence of interest and re-introduction into the gene as a cassette is also described by Loeb et al. (Genome 31(1):112, 1989) and Oliphant et al. (Gene 44(2-3):177, 1986)

US Patent 5,723,323 (1985) discloses a method for saturation mutagenesis at specific sites in a sequence by use of synthetic polynucleotide coupling. The resulting, stochastically generated polynucleotide sequences are subsequently introduced into vectors containing the gene of interest.
In a particular mode of carrying out this process, stochastic genes are produced by stochastic copolymerization of the four kinds of deoxyphosphonucleotides, A, C, G and T from the two ends of an initially linearized expression vector, followed by formation of cohesive ends in such a fashion as to form a stochastic first strand of DNA constituted by a molecule of expression vector possessing two stochastic sequences whose 3' ends are complementary, followed by the synthesis of the second strand of the stochastic DNA.

Hermes et al. (Gene 84(1):143, 1989; Proc.Natl.Acad.Sci.USA 87(2): 696, 1990) describe a method to randomize larger parts of a gene by use of so-called "spiked" oligodeoxyribonucleotide primers. The method was developed for the random mutagenesis of the gene for triosephosphate isomerase. By providing oligonucleotides containing a certain percentage of the non-matching bases at every position, a library of mutants was produced with the mutations restricted to those sequence parts that are defined by the primer binding sites.

Lanio and Jeltsch (Biotechniques. 25(6):958, 1998) describe another approach with mutagenic primer oligonucleotides to randomize selected parts of a gene with the wildtype being excluded from the transformants. With the mutagenized site being used as the cloning site, modified clones can efficiently be isolated after the mutagenesis step.

In summary, all the above-mentioned random mutagenesis methods can be classified by their requirement for sequence information. A first set of methods is directed to randomization of polynucleotides that comprise entire genes, genomes or parts of genes, and, therefore, do not require the underlying sequence information. However, these methods do not teach any possibility of introducing mutations limited to sites that are relevant or essential for the function or phenotype of the gene product encoded by the polynucleotide or that have been arbitrarily selected by the experimentator. A second set of methods, on the other hand, are directed to randomization of particular sites in a polynucleotide sequence. These methods range from randomization of single, specific positions to the randomization of entire regions. All these methods do, however, require knowledge of the sequence information at the site to be mutagenized. This sequence information is then, for example, used to synthesize mutagenic primers that bind at these sites, or to synthesize oligonucleotide cassettes with a definable degree of mutations to be inserted at these sites by use of restriction enzymes that cut specifically at or next to these sites. Also, these methods are not applicable if a number of sites separated from each other in a polynucleotide sequence are to be randomized simultaneously, if the sites to be randomized are not fixed but change during a set of engineering experiments, or if there is no efficient possibility do determine the sequence of the target polynucleotides and to identify therein explicitly the relevant or essential sites.

It would, therefore, be advantageous to have a random mutagenesis method that enables the efficient randomization of sites without the requirement for sequence information on the target polynucleotides. It would be particularly advantageous to have a random mutagenesis method that enables the randomization of relevant or essential sites within a target polynucleotide without the requirement for prior explicit identification of these sites. Relevant or essential sites in a polynucleotide are easily and efficiently identified by comparison of two or more polynucleotides and selection of the sites at which these two or more polynucleotides differ. Therefore, it would be particularly advantageous to have a random mutagenesis method that enables the randomization of sites at or in proximity to those positions at which two or more polynucleotide sequences differ from each other. Methods with the aforementioned characteristics have not heretofore been available.

### Summary of the Invention

The technical problem underlying the present invention is to provide a method that enables the efficient randomization of sites without the requirement for sequence information on the target polynucleotides. A particular aspect of the technical problem underlying the present invention is to provide a method for the selective randomization of polynucleotides at relevant or essential sites without requiring the explicit knowledge of these sites. This technical problem has been solved by providing the embodiments characterized in the claims.

Therefore, the present invention is directed to a method for the randomization of polynucleotides at relevant or essential sites. These sites are defined by positions at which two or more polynucleotides differ from each other. The randomization provides polynucleotide populations that encode a diversity of phenotypes, whereby the diversity is restricted to relevant or essential sites or to the proximity of relevant or essential sites. The method comprises the steps
providing polynucleotides that differ at one or more sites from each other, whereby these differing sites define the sites that are to be randomized;
generating heteroduplices from these polynucleotides;
recognizing the resulting differing site(s);
selectively randomizing the polynucleotides at or in proximity to these differing sites.

Furthermore, the present invention is directed to a method for altering polynucleotide characteristics by combination of the randomization of polynucleotides according to steps (i) to (iv) as described above with the selection or screening of these polynucleotides or of the corresponding gene products. The invention is also directed to a method for altering polynucleotide characteristics by combination of the randomization of polynucleotides according to steps (i) to (iv) as described above with other random mutagenesis techniques such as mutagenic PCR or cassette mutagenesis and/or with in-vitro recombination techniques such as the method disclosed in WO 01/34835 and/or with the selection or screening of these polynucleotides or of the corresponding gene products.

In a first aspect of the invention, the method is directed to saturation mutagenesis of polynucleotides at positions that are characterized by mutations in an original polynucleotide sequence, whereby these mutations are generated in a preceding process that comprises subjecting the original polynucleotide to a homogeneous random mutagenesis method and selecting or screening those polynucleotide variants that have desired characteristics. Homogeneous random mutagenesis techniques typically have a bias toward a subset of all possible mutations. Accordingly, a combination of homogeneous random mutagenesis techniques with selection or screening steps can result in the selection of mutations that are only partially optimal for the gene product. When making use of the invention according to the first aspect, these pre-selected positions are randomized completely, i.e. any of the naturally occuring nucleotide is introduced at these positions, thereby enabling to select from the resulting focussed library with high efficiency variants with the optimal mutation.

In a second aspect of the invention, the method is directed to randomization of polynucleotides at regions that are characterized by mutations in these regions in an original polynucleotide sequence, whereby the mutations are generated in a preceding process that comprises subjecting the original polynucleotide to a homogeneous random mutagenesis method and selecting or screening those polynucleotide variants that have desired characteristics. When intending to engineer polypeptides by means of random mutagenesis techniques there is often the problem, that these mutagenesis techniques only exchange single nucleotides while the mutagenesis of one amino acid to any other amino acid to a certain extent requires the exchange of two or even three nucleotides in the particular codon. However, the probability of exchanging two or even three nucleotides in a particular codon by means of homogeneous random mutagenesis techniques is relatively low. When making use of the invention according to the second aspect, regions that can be identified as being relevant by identification of at least partially improving mutations in these regions via a pre-selection step are randomized specifically, thereby enabling to select from the resulting focussed library with high efficiency variants with the optimal mutation. These regions can have a size of a codon, i.e. three nucleotides, or can be larger, up to 30 or more nucleotides.

In a third aspect of the invention, the method is directed to randomization of polynucleotides at sites that correspond to codons in a polypeptide that have been identified as being tolerant to the exchange for codons encoding a specific amino acid. When intending to engineer polypeptides by means of random mutagenesis techniques there is often the problem, that a significant fraction of the randomized polynucleotides have no function at all, for example because the particular amino acid residue is necessary for the structure or for the folding mechanism of the polypeptide. When making use of the invention according to the third aspect, codons that can be identified as being exchangeable can selectively be randomized. For example, after every codon in a polynucleotide is exchanged for nucleotides coding for an alanine, all variants still encoding functional polypeptides are used as the starting polynucleotides in step (i) of the method of the invention as described above. This decreases the complexity to be screened significantly, thereby increasing the efficiency of engineering polypeptides by means of random mutagenesis drastically.

In a fourth aspect of the invention, the method is directed to randomization of polynucleotides at sites at which naturally occurring polynucleotides differ from each other. Analogous or related genes from the same or from different species are often highly homologous, having sometimes more than 90% homology at the nucleotide level. When making use of the invention according to the fourth aspect, polynucleotide populations can efficiently be generated where the mutagenesis is restricted to those sites at which such homologous genes are different, without determination of the sequence of these naturally occurring, homologous genes.

In a fifth aspect of the invention, the method is directed to the efficient randomization of polynucleotides at several, pre-defined sites simultaneously. It has been a significant problem to generate populations of polynucleotides being randomized at several regions or positions that are distributed over a large sequence such as a gene encoding a polypeptide, an operon encoding a metabolic pathway, or an entire genome. When making use of the invention according to the fifth aspect, regions that are known as being relevant can efficiently be randomized by providing in step (i) as described above two or more polynucleotides whose sequences differ at these particular sites from each other.

For example, two or more immunglobulin-encoding polynucleotides are provided that have the same sequence and differ only in the complementarity-determining regions (CDRs) of the heavy and the light chain, leading to a population of polynucleotides that are randomized specifically at the CDRs.
The following detailed description describes the preferred features, advantages and the utility of the present invention. The following drawings are provided in order to explain further the present invention in supplement to the detailed description:

### Brief Description of the Drawings

Figure 1 depicts schematically and exemplarily the method of the invention.
Figure 2 shows a first embodiment of the invention, wherein a single position is randomized.
Figure 3 shows a second embodiment of the invention wherein several nucleotides are removed in 3' direction.
Figure 4 shows a third embodiment of the invention, wherein regions are randomized at and in proximity in both directions to the differing site.

### Detailed Description of the Invention

In the framework of this invention the following terms and definitions are used.
The term "polynucleotide" corresponds to any genetic material of any length and any sequence, comprising single-stranded and double-stranded DNA and RNA molecules, including regulatory elements, structural genes, groups of genes, plasmids, whole genomes, and fragments thereof. The term "site" in a polynucleotide refers to a certain position or region in the sequence of the polynucleotide. The term "position" in a polynucleotide refers to specific single bases in the sequence of the polynucleotide. The term "region" in a polynucleotide refers to stretches of several bases in the sequence of the polynucleotide. The term "differing site" is defined as at least one nucleotide which do not form a A/T or G/C Watson-Crick base pairing.The term "polypeptide" comprises proteins such as enzymes, antibodies and the like, medium-length polypeptides such as peptide inhibitors, cytokines and the like, as well as short peptides down to a amino acid sequence length below ten, such as peptidic receptor ligands, peptide hormones, and the like.The term "gene product" corresponds to any product, including, but not being limited to, polypeptides, that is encoded by a polynucleotide and that has a particular phenotype being selectable by any means of screening or selection technique. The terms " random mutagenesis" or "randomization" as used in this description indicate the manipulation of polynucleotides by unpredicted, stochastical replacements of the original nucleotide at a position with any other nucleotide. Alternatively, the term can also indicate the manipulation of polypeptide sequences by unpredicted, stochastical replacements of the original amino acid residue at a position with any other amino acid residue. Randomization or random mutagenesis methods usually lead to populations of polynucleotides or polypeptides that are related but differ from each other in one or more positions. "Heteroduplices" refer to double-stranded polynucleotide molecules comprised of single strands that differ at one or more positions from each other. If two single-stranded polynucleotides that differ in one or more positions are annealed, the resulting double stranded heteroduplex comprises base-paired and non base-paired regions. In DNA, adenine (A) usually pairs with thymidine (T) and guanine (G) usually pairs with cytosine (C). All other combinations usually do not form base-pairs and are therefore termed "mismatches". "Nicks" are incisions in the backbone of a double-stranded polynucleotide in one of either strands. These single-stranded breaks can be generated by an agent that is able to introduce nicks into a double-stranded polynucleotide. "Nucleobases" or "bases" are abbreviated as given in Table 2.

**Table 2:**

| **Abbreviation** | **Nucleobase** |
|---|---|
| A | Adenine |
| C | Cytosine |
| G | Guanine |
| T | Thymidine |
| U | Uracile |
| N | A, C, G, T, or U |
| V | Universal bases |
| - | AP site (position with the base being removed from the backbone) |
| X | Mutation (position at which two or more polynucleotides differ from each other) |

The term "universal base" refers to base analogs that are able to pair with more than one of the naturally occuring bases. Analogously, the term "universal nucleotide" refers to nucleotide analogs that can be incorporated into polynucleotides and after incorporation are able to pair with more than one of the naturally occuring nucleotides.

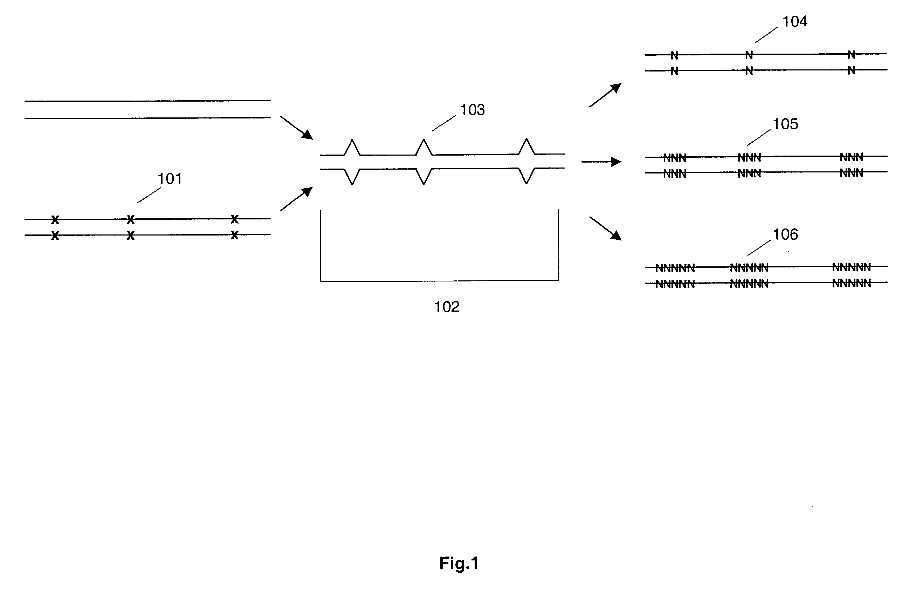

The principle of the present invention is schematically and exemplarily shown in Fig. 1. The method is directed to the randomization of polynucleotides at relevant or essential sites. These sites are defined by positions at which two or more polynucleotides differ from each other. The randomization provides polynucleotide populations that encode a diversity of phenotypes, whereby the diversity is restricted to relevant or essential sites or to the proximity of these sites. The method comprises the provision of polynucleotides that differ at one or more sites from each other (101, mutations indicated with an "X"), the generation of heteroduplices from these polynucleotides (102), and the recognition and selective randomization of the resulting mismatches (103, randomized positions indicated with an "N"), either focussed to a single mismatching nucleotide (104), or to a codon of three nucleotides (105), or to a region or a larger stretch of surrounding nucleotides (106).

In a preferred embodiment, the method comprises the following steps
providing polynucleotides that differ at one or more sites from each other, whereby these one or more differing sites define the sites that are to be randomized;
generating heteroduplices from the polynucleotides provided in step (a) leading to mismatches at the one or more sites;
introducing single-strand nicks at one or more of the mismatches generated in step (b), by means of an agent that is able to specifically recognize mismatch sites;
removing one or more nucleotides from the polynucleotide heteroduplex starting at the single-strand nicks generated in step (c);
filling the one or more gaps produced in step (d) under conditions that lead to the incorporation of one or more mismatching nucleotides, thereby randomizing the polynucleotides specifically at relevant or essential sites.

In a particularly preferred embodiment, steps (c) and (d) are executed simultaneously, i.e. mismatching nucleotides are removed directly in one step. Alternatively, the nucleobase of the mismatching nucleotide is removed simultaneously with the introduction of the single-strand break, thereby leading to an apurinic /apyrimidinic (AP) site, which is afterwards modified to lead to an extendable 3'-OH end. In another particularly preferred embodiment, this single nucleotide gap is extended further 5'- 3', 3'-5' or in both directions simultaneously. In another particularly preferred embodiment, the filling of the gap according to step (e) leads to a nick at the end of the polymerized stretch of nucleotides, which is then covalently closed by means of a ligase enzyme, optionally in combination with a polynucleotide kinase. In another particularly preferred embodiment, there is no gap formed, but instead steps (d) and (e) are executed simultaneously, i.e. nucleotides next to the nick introduced in step (c) are removed simultaneously to the incorporation of one or more mismatching nucleotides. The remaining nick is preferably covalently closed by means of a ligase enzyme. As an alternative, after incorporation of one or more mismatching nucleotides, the polymerization conditions are switched to non-mutagenic conditions, and the strand is synthesized without incorporation of mismatching nucleotides.

Starting material for the method of the invention are two or more polynucleotides that differ at one or more sites from each other. These differences mark the sites where randomization is performed. These polynucleotides are preferably provided as linear PCR products, either in a single-stranded or in double-stranded form. Alternatively, other linear polynucleotides, such as linearized plasmids or parts of a gene can be used analogously. When starting with two polynucleotides, these polynucleotides are preferably provided in a single-stranded form, one as the plus and one as the minus strand, thereby enabling the selective generation of heteroduplices. When starting with more than two polynucleotides, these polynucleotides are preferably provided in a double-stranded form in order to allow every possible heteroduplex pair be formed. The fraction of homoduplices, that per definition do not contribute to the further random mutagenesis process, decreases when increasing the number of double-stranded polynucleotides provided. For example, if two polynucleotides are provided at the same concentrations, the fraction of homoduplices is on average 50%, whereas, if twenty polynucleotides are provided at equal concentrations, the fraction of homoduplices is on average 5%.

The polynucleotides provided in step (a) can originate from different sources. They can originate from the preceding randomization of an original polynucleotide combined with one or more selection or screening steps that select those polynucleotides that encode gene products with improved characteristics. Preferably, the preceding randomization is done homogeneously, leading to mutations over the entire polynucleotide. Furthermore, starting polynucleotides can originate from the scanning of an original polynucleotide for sites - comprising single positions or longer regions - that are tolerant for a nucleotide exchange in the polynucleotide and/or for an amino acid exchange in the encoded polypeptide. Alternatively, starting polynucleotides are analogous or related genes or parts thereof isolated from the same or different species, showing a minimum degree of homology. As a further alternative, the positions at which polynucleotides differ can be introduced arbitrarily in order to provide marked polynucleotides to be selectively and efficiently randomized at these positions. The polynucleotide can have a length in the range between a few nucleotides and up to several kilobases. Preferably, polynucleotides are between 10 and 100,000 nucleotides long, more preferably between 100 and 10,000 nucleotides, and most preferably between 500 and 5,000 nucleotides.

In step (b), heteroduplices are generated from the polynucleotides provided in step (a). If the starting materials are double-stranded polynucleotides, the polynucleotides are mixed, then subjected to conditions that lead to melting of the double-strands to produce single-stranded molecules, which is followed by reannealing of these single strandes (Current Protocols in Molecular Biology, 1987-1988, Wiley Interscience). If the starting materials are single-stranded polynucleotides, those are mixed and randomly annealed to form double-stranded polynucleotides. The resulting heteroduplex molecules comprise mismatches, which can selectively be targeted by chemical, biochemical and/or enzymatic means.

In step (c), nicks are introduced into the heteroduplices specifically at or directly next to the mismatch sites. Such a nick is either a sole single-strand break in the phosphodiester backbone at the 5' or 3' side of the mismatch site, or the removal of the entire mismatching nucleotide, or the removal of several nucleotides at or around the particular mismatch site. The introduction of nicks is usually random with respect to the particular strand in the heteroduplex to be nicked. In particular embodiments, however, one of the two strands can be selectively nicked, thereby increasing the possible frequency of randomized sites per polynucleotide in the resulting populations.

Single-strand breaks at mismatch positions can be produced by several enzymatic and non-enzymatic ways. Vsr endonuclease from *E. coli* is particularly useful. The enzyme cleaves double-stranded DNA at T:G base-pair mismatches and produces a single-strand break 5' to the incorrectly paired T with a free 3'-OH and a 5'-phosphate residue at this nick. The enzyme shows a preference for T:G mismatches within a particular sequence context. The consensus sequence is N₁T^{A}/_{T}GN₂. N stands for A, T, G or C, the underlined T is opposed by a dG base (Gläsner, W. *et al*., J.Mol.Biol. 245(1):1, 1995; Lieb,M. and Rehmat,S., J.Bacteriol. 177(3):660,1995). Another useful enzyme is the *E. coli* endonuclease IV. This enzyme is a class II AP endonuclease with 3'-repair phosphodiesterase activity cleaves the phosphodiester backbone on the 5'side of the apurinic/apyrimidinic (AP) sites leaving a 5'-terminal 2-deoxyribose 5-phosphate residue (dRP, removable by dRPase activity) and a free 3'-OH residue. The enzyme removes 3' blocking fragments, e.g. phosphoglycoaldehyde, deoxyribose-5-phosphate, 4-hydroxy-2-pentenal, and phosphate groups from the 3'ends of DNA left by AP lyase activity (Friedberg,E.C.*et al.,* DNA Repair and Mutagenesis, ASM Press, Washington: 157-158, 1995; Levin,J.D. *et al*., J.Biol.Chem. 266(34): 22893, 1991). *E. coli* Endonuclease V (deoxyinosine 3'-endonuclease) is another useful enzyme. It recognizes mismatches in duplex DNA and cleaves the second and third phosphodiester bonds 3' to the mismatch at 95% and 5% frequency, respectively. The enzyme produces a nick with 3'-hydroxyl and 5'-phosphoryl groups in the strand with the mismatch closest to the 5'end. Unlike the members of the glycosylase-class of enzymes endonuclease V does not appear to release free bases from DNA. Another particularly useful enzyme is Endonuclease V, which cleaves DNA duplexes containing AP sites, urea residues, hairpin or unpaired loops, flaps, and pseudo-Y structures. (Yao,M. *et al*., J.Biol.Chem., 269(23): 16260, 1994). The mode of action of the enzyme depends on the reaction conditions, i.e. pH, presence of MnCl₂ or MgCl₂. A further enzyme performing incision on the 3'- side of the mismatch site in one of the two DNA strands in a heteroduplex is the CEL I-Iike nuclease isolated from celery (Oleykowski et al. Nucleic Acids Res. 26(20): 4597, 1998).
A further, particularly useful enzyme in this context is MutY. The enyzme is a bifunctional glycosylase. It recognizes A/G and A/8-oxo-dG mismatches in duplex DNA and cleaves the strand containing the A. The opposite strand is not cleaved. MutY has an associated AP lyase activity (Lu,A.L. and Hsu,I.C., Genomics 14(2): 249,1992; Friedberg,E.C.*et al*., DNA Repair and Mutagenesis, ASM Press, Washington: 157-158, 1995). MUG from E. coli is a further useful enzyme. MUG removes pyrimidines uracil (deamination of cytosine) and thymine (deamination of 5-methylcytosine) from U/G and T/G mismatches ( Barrett,T.E *et al*., Cell 92(1):117, 1998; Barrett,T.E *et al*., EMBO.J. 18(23): 6599, 1999). TDG (Thymine mismatch DNA glycosylase, from M. thermoautotrophicum) is another particularly useful enzyme. TDG recognizes T/G (U/G, G/G, T/T, T/C) mismatches (deamination of 5'-methylcytosine to thymine) in dsDNA. TDG is a monofunctional glycosylase. The enzyme specifically removes thymine and uracil bases mispaired with guanine through hydrolysis of their N-glycosidic bond, thereby generating abasic sites in DNA. A further useful enzyme is Human endonuclease IV homolog APE/HAP1. The enzyme cleaves DNA at AP sites forming nicks in DNA (Yacoub,A. *et al*., Cancer.Res. 57(24): 5457, 1997; Duguid, J.R.*et al*., Cancer.Res., 55(24): 6097, 1995). In contrast to endonuclease IV, APE1 shows only weak 3'-repair diesterase activity on deoxyribose fragments located at DNA strand breaks Demple,B and Harrison,L., Annu.Rev.Biochem. 63:915, 1994; Xu,Y.J *et al*., J.Biol.Chem. 273(44): 28837,1998). A further useful enzyme is E. coli exonuclease III. This enzyme has a class III AP endonuclease activity besides the 3'- to 5'-exonuclease activity. It acts on 3'-OH, 3'-phosphate, and 3'-phosphoglycolate groups (Friedberg,E.C.*et al*., DNA Repair and Mutagenesis, ASM Press, Washington: 157-158, 1995).

As an alternative to enzymatic processes, single-strand breaks at mismatch positions can also be produced by chemical cleavage (CMC-chemical mismatch cleavage). Osmiumtetroxide, potassium permanganate is known to recognise and modify a range of mismatched bases (T/C, T/G, T/T and C/T, C/A, C/C mismatches). Potassium permanganate/ tetraethylammonium chloride and hydroxylamine are next to others further alternatives (Roberts,E. *et al*., Nucleic.Acids.Res. 25(16): 3377, 1997).

The removal of single-strands according to step (d) can be limited to several nucleotides to generate single-strand regions in proximity to the mismatch positions within the double-stranded polynucleotides. Alternatively, the removal of the single-strands according to step (d) can be unrestricted, thereby extending the gap from the mismatch positions to the end of the polynucleotides. Exonucleases and polymerases can be advantageously used for this purpose. Examples of useful exonucleases are Lambda-exonuclease (5' □ 3'exonuclease) (Little, Gene Amplification & Analysis 2 (1981), 135-145); T7 exonuclease (5'→3'exonuclease), T5 D15 exonuclease (5'→3' exonuclease)( Sayers et al., J. Biol. Chem. 265 (1990), 18311-18317), 5'-3' exonuclease from the bacteriophage N4 (Guinta et al. (J. Biol. Chem. 261 (1986), 10736-10743), 5'-3'-exonuclease from nuclear extracts (Exol) from Saccharomyces cerevisiae (Huang and Symington (Mol. Cell. Biol. (1993), 3125-3134), Exonuclease III (3 '→5'exonuclease), Exonuclease I (3'→5'exonuclease) (Brody et al. (J. Biol. Chem. 261 (1986), 7136-7143), Brody and Doherty (Biochemistry 24 (1985), 2072-2076), YNT20 from Saccharomyces cerevisiae (3'-5' exonuclease) (Hanekamp and Thorsness (Current Genetics 34 (1999), 438-448), DNA-polymerase-lil-subunit-epsilon of E. coli (3'→5' exonuclease) (Krutyakov (Mol. Biol. 32 (1998), 197-199), Examples of useful polymerases are DNA polymerase I (5'→3'polymerase, 3'→5' and 5'→3' exonuclease) (Rigby et al., J. Mol. Biol. 113 (1977), 237-251), Taq (Tth) polymerase (5'→3'polymerase, 3'→5' and 5'→3'exonuclease)(Longley MJ et al.,Nucleic Acids Research. 18(24):7317-22, 1990), Klenow fragment (5'→3' polymerase, 3'→5' exonuclease) (Sanger, Proc. Natl.Acad. Sci. USA 74 (1977), 5463-5467), T4 DNA polymerase (5'→3'polymerase, 3'→5' exonuclease)(Young et al., Biochemistry 31(37): 8675,1992), Pwo, Pfu, Pfx, Tub, Vent, Tma, UITma polymerases (5 '→3'polymerase, 3'→5' exonuclease).(Newton and Graham, in: PCR, Spektrum Akad. Verlag Heidelberg (1994), 1))

The filling of the gaps according to step (e) is carried out by polymerization of nucleotides. Preferably, the filling can be done with a standard polymerase under conditions that lead to an increased frequency of misincorporations (e.g. conditions of mutagenic PCR as described by Cadwell,R.C and Joyce,G.F., PCR Methods Appl. 2 (1): 28, 1992; PCR Methods Appl. 3 (6): 136, 1994).
More preferably, the filling of the gaps can be carried out with a polymerase and universal nucleotides. Universal nucleotides are characterized as being able to form basepairs alternatively with two or more of the four standard nucleobases;
Therefore, universal nucleosides are, but not limited to, dI (2'-deoxy-inosine), dP (P coding for 6H,8H-3,4-dihydropyrimido[4,5-c][1,2]oxazin-7-one, with "p" serving as pyrimidine (C or T) analogue, Lin and Brown, Nucleic Acids Research. 17(24):10373-83, 1989), dK (K coding for N6-methoxy-2,6-diaminopurine, with "K" serving as a purine (G or A) analogue, Lin and Brown, Nucleic Acids Research. 20(19):5149-52, 1992). Further, as universal bases can be used 3-nitropyrrole (Nichols et al., Nature, 369:492, 1994; Bergstrom et al., J. Am. Chem. Soc, 117: 1201, 1995) or 4-, 5-, and 6- nitroindole (Loakes et al, Nucleic Acids Research. 22(20):4039-43, 1994).
Alternatively, the filling of the gaps according to step (e) can be carried out with a polymerase and unequal mixtures of the four standard nucleotides (dATP, dCTP, dGTP, dTTP). As further alternative, filling of the gaps can be carried out with a polymerase in four separate reactions, whereby in each reaction of the four standard nucleotides (dATP, dCTP, dGTP, dTTP) is lacking. Furthermore, filling of the gaps can be carried out with a polymerase and a mixture of standard (dATP, dCTP, dGTP, dTTP) and universal nucleotides such as dITP.
Dependent on the incorporation rate of each of the nucleotides, mixtures of unequal concentrations of each nucleotide are provided. By means, to enforce the integration of a nucleotide with lower incorporation affinity in comparison to the other, thus is to be provided in higher concentration.

As another alternative, filling of the gaps can be carried out with a polymerase and a mixture of random nucleotide trimers, with specific oligonucleotides generated from the original pool of genes but carrying mutations, with completely random oligonucleotides, or with a combination of these.
Further on, the filling of the gaps according to step (e) can be carried out with a ligase and specific and/or random oligonucleotides or mixtures thereof. Instead of modifying the conditions during polymerization, the polymerase can also be chosen to have a high error rate (Suzuki,M. et al., J.Biol.Chem. 272(17): 11228, 1997).

Polynucleotides generated in step(e) can be subjected to amplification procedures.
In vitro PCR amplification is performed under conditions offering any of the standard nucleotides dNTPs. Preferably, the amplification is carried out with unequal mixtures of the four standard nucleotides in order to compensate any bias for the nucleotide incorporated opposite to an universal nucleotide during the amplification. Polynucleotides obtained in step (e) can also be amplified in vivo.

In a first embodiment of the method of the invention the degradation of the nicked strand according to step (d) is limited to one nucleotide to generate an unpaired nucleotide only at the specific mismatching positions.

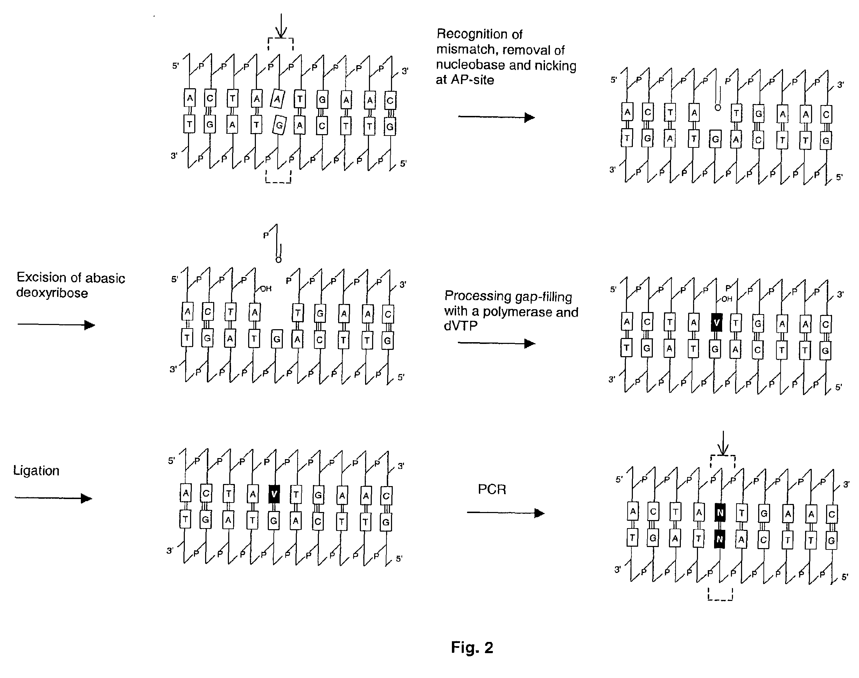

A particularly preferred variant of this embodiment is depicted in Fig. 2. According to this variant, the mismatching nucleobase is first removed by an agent that is able to specifically recognize mismatches and that has DNA glycosylyase and AP lyase activity. The resulting nicked abasic deoxyribose moiety is preferably removed by an agent having AP endonuclease activity such as Endonuclease IV from *E.coli* (Friedberg,E.C.*et al*., DNA Repair and Mutagenesis, ASM Press, Washington: 157-158, 1995; Levin,J.D. *et al*., J.Biol.Chem. 266(34): 22893, 1991), human Endonuclease IV (Yacoub,A. *et al*., Cancer.Res. 57(24): 5457, 1997; Duguid, J.R.*et al*., Cancer.Res., 55(24): 6097, 1995 ), Exonuclease III from *E.coli* (Friedberg,E.C.*et al*., DNA Repair and Mutagenesis, ASM Press, Washington: 157-158, 1995), leading finally to a single-nucleotide gap having an extendable 3'-OH at the position of the former mismatch. This embodiment can be followed by the introduction of a single, universal nucleotide, such as dITP, by means of a polymerase, and ligation of the resulting nick by means of a ligase enzyme, optionally combined with a polynucleotide kinase.

PCR amplification of this modified polynucleotide or amplification by inserting in a vector and transformation into a cell lead finally to a population of polynucleotide molecules comprising random mutations specifically at the mismatching position.

In a second embodiment of the invention, the removal of nucleotides from the nicked strand according to step (d) is done simultaneously to the incorporation of new nucleotides according to step (e) by means of a polymerase having 5'-3' exonucleolytic activity or strand displacement activity to randomize positions at the 3' side of the mismatching positions.

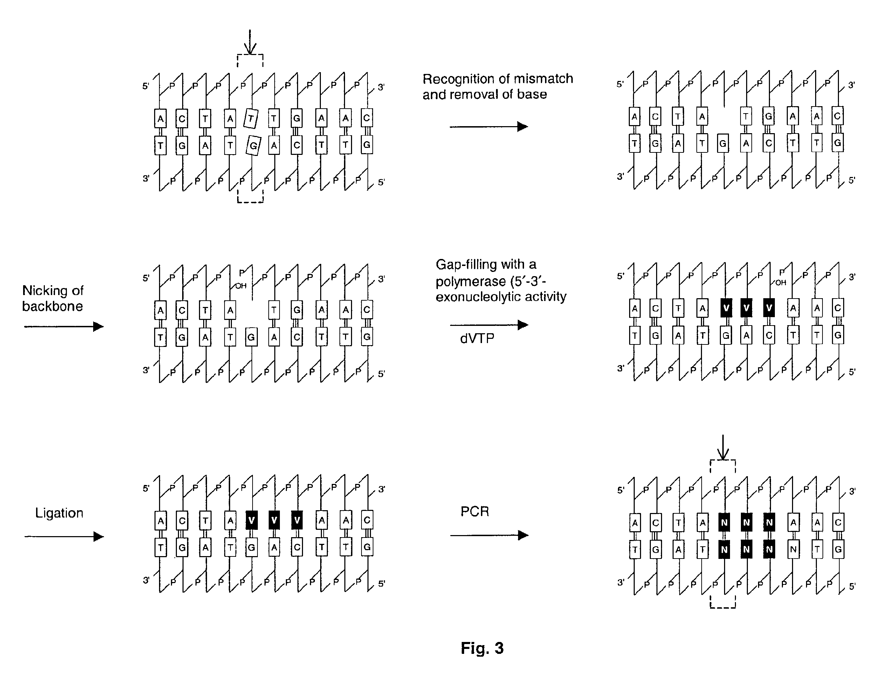

A particularly preferred variant of this second embodiment is depicted in Fig. 3. According to this variant, the mismatching nucleobase is first removed by an agent that is able to recognize mismatches and to excise the corresponding nucleobase resulting in an AP site. Preferably, an enzyme with DNA glycosylase function such as TDG (Thymine mismatch DNA glycosylase from *M. thermoautotrophicum*, Neddermann,P. et al., J.Biol.Chem. 271(22): 12767, 1996) or MUG (Mismatch uracil DNA glycosylase from *E.coli*, Barrett,T.E *et al*., Cell 92(1):117, 1998; Barrett,T.E *et al*., EMBO.J. 18(23): 6599, 1999) is used for this step. The phosphodiester bond 5' of the AP site is then hydrolyzed by means of a second agent leading to an extendable 3' OH end. Preferably an enzyme having AP endonuclease function such as E.coli Endonuclease IV (Friedberg,E.C. *et al*., DNA Repair and Mutagenesis, ASM Press, Washington: 157-158, 1995; Levin,J.D. *et al*., J.Biol.Chem. 266(34): 22893, 1991) or human Endonuclease IV (Yacoub,A. *et al*., Cancer.Res. 57(24): 5457, 1997; Duguid, J.R.*et al*., Cancer.Res., 55(24): 6097, 1995) is used for this step. The resulting 3' OH end is then extended by means of a polymerase optionally having dRPase (deoxyribose phosphatase) function in order to remove the remaining abasic deoxyribose phosphate moiety, as e.g. Human DNA polymerase β (Matsumoto et al., Science 269(5224):699, 1995), *Drosophila* ribosomal protein S3 (Sandigursky et al. J.Biol.Chem. 272(28): 17480,1997). Particularly useful polymerases with 5'-3'-exonucleolytic activity for the removal of nucleotides during the incorporation of new nucleotides are DNA polymerase I (Rigby et al., J. Mol. Biol. 113 (1977), 237-251) or Taq polymerase from Thermus aquaticus . Particularly useful polymerases with strand-displacement activity for the removal of nucleotides during the incorporation of new nucleotides are DNA polymerase δ, large fragments of rBst DNA polymerase from B. stearothermophilus, Phi29 DNA polymerase (Giesler et al., Amersham Pharma Biotech). If a polymerase with strand-displacement activity is used the displaced single-strand has to be cleaved by means of a DNase IV or mammalian FEN-1 or Rad27 from *Saccharomyces cerevisiae* (Negritto et al., Molecular and Cellular Biology 21(7):2349, 2001). Incorporated nucleotides are either universal bases (such as dITP, dPTP, dKTP) or standard nucleotides under conditions that lead to an increased misincoporation rate. After ligation of the resulting nick by means of a ligase enzyme, optionally combined with a polynucleotide kinase, the polynucleotides are either PCR-amplified or amplified by inserting into a vector and transformation into a cell lead finally to a population of polynucleotide molecules comprising random mutations specifically 3' downstream from the mismatching position. In another variant of this preferred embodiment randomization can be done by a first polymerization step under conditions that lead to a high frequency of misincorporation and a second polymerization step under conditions that lead to a low frequency of misincorporation. In particular, the first polymerization step is carried out with a polymerase having 5'-3' exonucleolytic activity and using universal nucleotides. The second polymerization step is then carried out with a polymerase having 5'-3' exonucleolytic activity and using standard nucleotides. As an alternative to the aforementioned variants, a polymerase with DRPase but without 5'-3'-exonucleolytic activity and strand-displacement activity can be used. Then only a single nucleotide is incorporated leading to the same result as the first embodiment.

In a third embodiment of the invention, the removal of nucleotides from the nicked strand according to step (d) is done by means of an exonuclease thereby allowing to randomize a region extending from the mismatch site either to the 3' side, or to the 5' side, or to both, the 3' and the 5' side. The size of this region is preferably confined by controlling the exonucleolytic digestion.

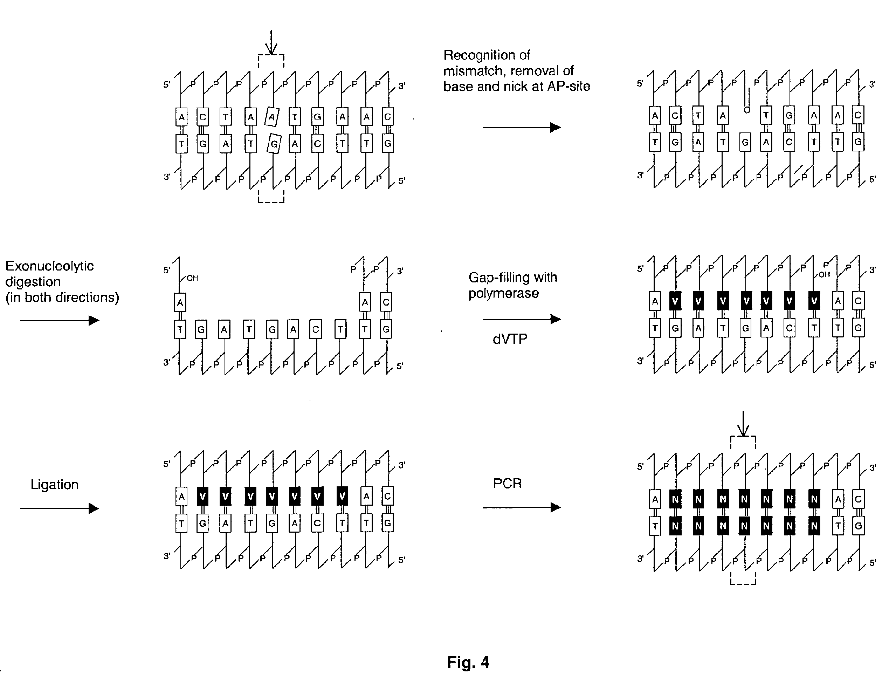

A particularly preferred variant of this third embodiment is depicted in Fig. 4. According to this variant, the mismatching nucleobase is first removed by an agent that is able to specifically recognize mismatches and that has DNA glycosylase and AP lyase activity. The resulting nicked abasic deoxyribose moiety can optionally be removed by an agent having AP endonuclease activity such as E.coli Endonuclease IV (Friedberg *et al*., *1995*), Human Endonuclease IV (Yacoub et al.,1997 ). The nick is then extended to a gap of a certain size by means of an enzyme having exonuclease activity. In a particularly preferred embodiment the gap is extended in 3' direction by means of an exonuclease that specifically has single-strand 3'-5'-exonucleolytic activity such as Exonuclease III from *E.coli* ( Friedberg *et al*., *1995*) or *E.coli* Exonuclease I (Brody et al. J. Biol. Chem. 261: 7136 ,1986). In another particularly preferred embodiment the gap is extended in 5' direction by means of an exonuclease that specifically has single-strand 5'-3'-exonucleolytic activity such as λ -Exonuclease or T7-5'-exonuclease derived from the bacteriophage T7. In a further, particularly preferred embodiment the gap is extended in both directions by means of an exonuclease that has single-strand 3'-5'- and 5'-3'-exonucleolytic activity such as Bal 31 from Alteromonas espejiana (Gray et al., Nucleic Acid Res. 2 (1975) 1459-1492) or by means of a blend of enzymes having single-strand 3'-5'-exonucleolytic and 5'-3'-exonucleolytic activity. The resulting 3' OH end is then extended by means of a polymerase lacking 5'-3'-exonucleolytic and strand-displacement activity. Particularly useful polymerases for this purpose are T7 DNA polymerase, Klenow fragement, T4 DNA polymerase. Incorporated nucleotides are either universal bases such as dITP or standard nucleotides under conditions that lead to an increased misincoporation rate.

In another variant of this preferred embodiment randomised oligonucleotides of different length are being hybridised to the gaps of ssDNA generated as outlined above. Optionally, these olignucleotides may contain varying degrees of universal bases.
After ligation of the resulting nick by means of a ligase enzyme, optionally combined with a polynucleotide kinase, the polynucleotides are either PCR-amplified or amplified by inserting into a vector and transformation into a cell leading finally to a population of polynucleotide molecules comprising random mutations specifically 3' downstream or 5' upstream or to both direction from the former mismatching position.

Several combinations of the above described embodiments can be defined leading to particular useful variants of the method of the invention. It is understood that the embodiments described herein are for illustrative purposes only and that various modifications or changes in light thereof will be suggested to persons skilled in the art and are to included within the spirit and purview of this application and are considered within the scope of the appended claims. All publications, patents, and patent applications cited herein are hereby incorporated by reference in their entirety for all purposes.

### Experimental Section:

### Example 1: Generation of DNA-Heteroduplices

The following single-stranded polynucleotides were used to generate double-stranded polynucleotides with homologous and heterologous regions.

Polynucleotide 1 (SEQ ID NO:1):
5'CGTTGCATATGTGGAAGAAGATCATATTGCACATGAATATGCACAGAGTGTTCCTTAT
GGCATTTCTCAAATTAAAGCGCCGGCTCTTCACTCTCAAGGCTACACAGGCTCTAACG

Polynucleotide 2 (SEQ ID NO:2):
5'CGTTGCATATGTGGAAGAAGATCATATTGCACATGAATATGCACAGAGTGCTCCTTAT
GGCATTTCTCAAATTAAAGCGCCGGCTCTTCACTCTCAAGGCTACACAGGCTCTAACG

Polynucleotide 3 (SEQ ID NO:3):
5'CGTTGCATATGTGGAAGAAGATCATATTGCACATGAATATGCACAGAGTGTTCCTTAT
GGCATTTCTCAAATTAAAGCGCCGGCTCTTCACTCTCAAGGCTACACAGGCTCTAACGT
AAAAGTAGCTGTTATCGACAGCGGAATTGACTCTTCTCATCCTGACTTAAACGTCAGAG
GCGGAGCAAGCTTCGTACCTTCTGAAACAAACCCATACCAGGACGGCAGTTCTCACGGT
ACGCATGTAGCCGGTACGATTGCCGCTCTTAATAACTCAATCGGTGTTCTGGGCGTAGC
GCCAAGCGCATCATTATATGCAGTAAAAGTGCTTGATTCAACAGGAAGCGGCCAATATA
GCTGGATTATTAACGGCATTGAGTGGGCCATTTCCAACAATATGGATGTTATCAACATGA
GCCTTG

Polynucleotide 4 (SEQ ID NO:4):
5'CGTTGCATATGTGGAAGAAGATCATATTGCACATGAATATGCACAGAGTGCTCCTTAT
GGCATTTCTCAAATTAAAGCGCCGGCTCTTCACTCTCAAGGCTACACAGGCTCTAACGT
AAAAGTAGCTGTTATCGACAGCGGAATTGACTCTTCTCATCCTGACTTAAACGTCAGAG
GCGGAGCAAGCTTCGTACCTTCTGTAACAAACCCATACCAGGACAGCAGTTCTCACGGT
ACGCATGTAGCCGGTACGATTGTCGCTCTTAATAACTCAATCGGTGTTCTGGGCGTAGC

GCCAAGCGCATCATTATATGCAGTAAAAGTGCTTGATTCAACAGGAAGCGGCGTATATA
GCTGGATTATTAACGGCATTGAGTGGGCCATTTCCAACAATATGGATGTTATCAACATGA
GCCTTG

The polynucleotides 1 and 2 as well as 3 and 4 were mixed in aequimolar amounts to yield a solution of 1 µg DNA in 20 µl water. Annealing was performed by heating the solution in a PCR cycler to 94 °C and subsequent cooling with a rate of 0.04 °C/s to 50 °C. The single-stranded polynucleotides 1 and 2 create a double-stranded polynucleotide with a mismatch at position 51 (Heteroduplex 1). The single-stranded polynucleotides 3 and 4 create a resulting double-stranded polynucleotide with a variety of mismatches such as T/G, A/A, G/T, C/A, A/C, A/A at the positions 51, 201, 221, 258, 347, 348 respectively which comprise 3 of the 8 possible mismatches (Heteroduplex 2).

### Example 2: Introduction of single-stand nicks at mismatches

Mismatches in the heteroduplices are recognized by DNA-Glycosylases. In this example the following DNA-Glycosylases are used:
a.)TDG (Thymin-DNA-Glycosylase, which recognize mismatches in the order T/G >> T/C > T/T and cleaves specific the single-strand at T)
b.)MutY (MutY-DNA-Glycosylase, recognize A/G and A/C mismatches and cleaves specific the single-strand at A)

The analysis of the cleavage reaction was carried out with fluorescent-labeled heteroduplices each strand being labeled at its 5'end. Fluorescence labeled single-stranded polynucleotides were generated with 5'-end labeled primer and a standard PCR protocol. The respective PCR products were denatured and re-annealed under standard PCR conditions (94 °C → 50 °C, 0.04 °C/s) and purified (QIAgen PCR purification kit). These fluorescence labeled heteroduplices were submitted to enzymatic reactions. The resulting DNA fragments were analysed using a capillary electrophoresis system with fluorescence detection (CEQ 2000 DNA Analysis-System, Beckmann).

The addition of 1 µl TDG (2u/µl, R&D Systems) and 2 µl of 10 x TDG-Buffer (R&D Systems) to 20 µl (1 µg/20 µl) Heteroduplex 2 and incubation for 1h at 65 °C demonstrated specificity for T/G and T/T mismatches.

The addition of 1 µl (2 u/µl, R&D Systems) MutY to 0.5 µg Heteroduplex 2 in 50 µl REC-Buffer (R&D Systems) and subsequent incubation for 2h at 37 °C demonstrated the specificity of MutY for A/G mismatches.

### Example 3: Introduction of single-strand nicks at AP sites

Mismatches in heteroduplices can be recognized and modified by the cleavage of a nucleoside residue at one of the two mismatch basepairs. A double-stranded polynucleotide with an apurinic site (AP site) site can be cleaved by *E. coli* endonuclease IV under the following conditions: The double-stranded polynucleotide substrate with an apurinic site was generated by annealing (94°C → 50°C, 0.04 °C/s) oligonucleotide 1 (5'GAATATGCACAGAGTG[Sp-d]TCCTTATGGC) and oligonucleotide 2 (5'GCCATAAGGAGCACTCTGTGCATATTC). A total of 1 µg annealing product was incubated in 20 µl of TDG Buffer (R&D Systems) with 4 u endonuclease IV (E.coli, MBI Fermentas) for different periods of time. The reaction was stopped by adding 5 µl of 6 x loading buffer (MBI Fermentas) and boiling for 10 min at 95 °C. The reaction products were analysed using a 15% polyacrylamide gel and ethidiumbromide staining. There was an increase in intensity of the expected cleavage product with increasing time of the digestion.

### Example 4: Trimming of 3'-ends for polymerase reaction

Heteroduplex DNA displaying mismatches may be nicked by bi-functional DNA-Glycosylases which subsequent to glycosylase activity further incise at the 3' site via □β-elimination thereby producing an obstructive 3'end. These 3' blocking groups can be removed by *E. coli* endonuclease IV to generate suitable primers for extension reactions. Fragments generated by TDG action (Example 2) that had an obstructive 3'end were isolated from a denaturing PAGE gel employing standard procedures. In the following the blocked fragments were incubated with endonuclease IV using conditions as outlined in Example 3. The functionality of the trimmed oligonucleotide was demonstrated by primer extension under standard conditions. Reaction products were analysed as outlined in Example 2 and showed the extensibility of the endonuclease IV treated oligonucleotide.

### Example 5: Incorporation of dITP

A polynucleotide was generated by digesting labelled polynucleotide 3 with *Nae*I. The reaction products were purified (QIAgen Minelute) and annealed (94°C → 50 °C, 0.04 °C/s) with unlabelled polynucleotide 3 prior to elongation by Taq-DNA-Polymerase. The polymerase reactions were carried out under standard and mutagenic (PCR supplemented with 100 - 400 µM Mn2⁺) PCR protocol conditions in the absence of dNTP but in the presence of 2mM dITP. Elongation products were detected by DNA-fragment analysis described in example 2 under standard and mutagenic conditions demonstrating the incorporation of dIMPs.

### Example 6: Ligation of polynucleotides containing dIMP at the 3 '-end

Fluorescence labeled oligonucleotide 3 (5'Cy5-CGAGCGTTGC ATATGTGGAA GAAGATCATA TI) with a dIMP at the 3'-end was mixed with oligonucleotide 4 (5'[P]-GCACATGAAT ATGCACAGAG TGTTCCTTAT GGC) and unlabelled polynucleotide 3. After denaturation and annealing (94°C → 50°C, 0.04 °C/s), the oligonucleotides were ligated using 25 u T4-DNA-Ligase (MBI Fermentas) overnight at 16 °C under standard conditions. Ligation products of 65 nt single-stranded oligonucleotides were detected using the DNA-fragment analysis described in example 2.

### Example 7: Amplification of templates containing dIMP stretches

A standard PCR was performed using primers (oligonucleotide 5: 5'CGTTGCATAT GTGGAAGAAG and oligonucleotide 6: 5'CAAGGCTCATGTTGATAACATC) and a template according to polynucleotide 3 but containing a sequence of 8 dIMPs starting at position 205 for the forward template strand and no dIMP for the reverse template strand. After a standard PCR protocol, PCR products were analyzed quantitatively using standard absorption methods and scrutinized by means of DNA sequencing.

## Claims

1. A method for randomizing polynucleotides at specific sites which comprises providing at least one polynucleotide having at least one differing site and selectively randomizing the polynucleotides at or in the proximity to the at least one differing site.

2. The method of claim 1, wherein the polynucleotide is a double-stranded polynucleotide which is derived from at least one starting single-strand polynucleotide or is a heteroduplex generated from at least two polynucleotides that differ in at least one site from each other.

3. The method of claim 1 to 2, wherein the polynucleotides or their corresponding translational products are pre-selected with respect to their genotypic and/or phenotypic features.

4. The method of claim 1 to 3 which comprises the following steps:
(a) providing polynucleotides that differ at one or more sites from each other, whereby these differing sites define the start points for randomization;
(b) generating heteroduplices from these polynucleotides;
(c) recognizing the resulting differing sites;
(d) selectively randomizing the polynucleotide at or in proximity to these differing sites;

5. The method of claim 4 wherein steps (a) to (d), steps (a) to (b) and/or steps (c) to (d) are carried out for multiple cycle before entering into the next step.

6. The method of claim 1 to 5 wherein the differing sites of the polynucleotides consists of one or more mutation(s), preferably the mutations comprise
(i) one or more nucleotide substitution(s),
(ii) one or more nucleotide insertion(s),
(iii) one or more nucleotide deletion(s), or
(iv) a combination of (i) to (iii).

7. The method of claim 1 to 6 which further comprises selection or screening for at least one selectively randomized polynucleotide or its corresponding translational products towards a desired property.

8. The method of claim 1 to 7 which is carried out cyclically.

9. A method for randomizing polynucleotides at specific sites which comprises the following steps:
(a) providing polynucleotides that differ at one or more sites from each other, whereby these one or more differing sites specify the sites that are to be randomized;
(b) generating heteroduplices from the polynucleotides provided in step (a) leading to mismatches at the one or more sites;
(c) introducing single-strand nicks at one or more of the mismatches generated in step (b), by means of an agent that is able to specifically recognize mismatch sites;
(d) removing one or more nucleotides from the polynucleotide heteroduplex starting at the single-strand nicks generated in step (c);
(e) filling the one or more gaps produced in step (d) under conditions that lead to the incorporation of one or more mismatching nucleotides, thereby randomizing the polynucleotides at the specific sites.

10. A method for randomizing polynucleotides at specific sites which comprises the following steps:
(a) providing polynucleotides that differ at one or more sites from each other, whereby these one or more differing sites specify the sites that are to be randomized;
(b) generating heteroduplices from the polynucleotides provided in step (a) leading to mismatches at the one or more differing sites;
(c) introducing single-strand nicks at one or more of the mismatches generated in step (b), by means of an agent that is able to specifically recognize mismatch sites;
(d) removing one or more nucleotides from the polynucleotide heteroduplex starting at the single-strand nicks generated in step (c);
(e) filling the one or more gaps produced in step (d) at least in part with universal monomers, whereby universal monomers are characterized as being able to form basepairs alternatively with two or more of the four natural nucleobases;
(f) separating the heteroduplex strands from each other; and
(g) synthesizing the counter strands using the single strands generated in step
(f) as templates, thereby randomizing the polynucleotides specifically at sites where universal monomers were introduced in step (e).

11. The method according to claim 9 or 10 wherein
(i) the introduction of nicks in step (c) comprises the introduction of sole single-strand break in the phosphodiester backbone at the 3' or 5' side of the mismatching site, or the removal of the entire mismatch nucleotide, or the removal of several nucleotides at or around the mismatch site; and/or
(ii) the removal of nucleotides according to step (d) is either limited to several nucleotides to generate a single-strand region in proximity to the mismatch site, or is unrestricted to generate a gap from the mismatch position to the end of the polynucleotide; and/or
(iii) the removal of one or more nucleotides according to step (d) and with filling of the gap according to step (e) are carried out in parallel by means of a standard polymerase, a polymerase having 5'-3' exonuclease or strand displacement activity; and/or
(iv) the filling of the gap according to step (e) is carried out at least in part by use of oligonucleotides and a ligase enzyme.

12. The method of any one of the claims 9 to 11, wherein the filling of the gap according to step (e) is carried out with a polymerase and a mixture of 3 of the 4 standard nucleotides (dATP, dTTP, dGTP, dCTG), preferably the filling of the gap according to step (e) is carried out separately with different compositions of mixtures of 3 nucleotides (dATP, dTTP, dGTP, dCTG), and most preferably the separately filled gaps according to step (e) are pooled afterwards.

13. The method of any one of the claims 9 to 12, wherein the filling of the gap according to step (e) is carried out with a polymerase under highly mutagenic conditions or with a low-fidelity polymerase having a high error rate.

14. The method of any one of the claims 9 to 12, wherein the filling of the gap according to step (e) is carried out with a polymerase and dITP instead of dATP, dTTP, dGTP, dCTG or a mixture of dITP and dATP, dTTP, dGTP, dCTG in same or different concentrations.

15. The method of claim 1 which comprises providing variants of the polynucleotide sequence having at least one differing site and selectively randomizing the polynucleotide sequence at or in proximity to the differing site(s).

16. A method for optimizing polynucleotide sequence, comprising
providing variants from this polynucleotide sequence;
randomizing the polynucleotide sequence specifically at these sites at which these variants differ from each other;
the selecting or screening the randomized pool of polynucleotides for desired properties.

17. A method for optimizing a polynucleotide towards desired properties of its translational product which comprises
(a) introducing stochastically random mutations into polynucleotides;
(b) selecting or screening the population of polynucleotides generated in step (a);
(c) isolating those polynucleotides which encode gene products with improved characteristics;
(d) selectively randomizing the polynucleotides at or in proximity to those site(s), at which the polynucleotides isolated in step (c) differ from each other;
(e) selecting or screening the population of polynucleotides generated in step (d)
(f) isolating those polynucleotides which encode gene products with further improved characteristics,
in the above method steps (a) to (c) and/or steps (d) to (f), and/or steps (a) to (f) are preferably repeated iteratively.


SEQUENCE LISTING

<110> DIREVO Biotech AG

<120> Method for the Selective Randomization of
      Polynucleotides

<130> 022258ep/JH

<140>
<141>

<160> 4

<170> PatentIn Ver. 2.1

<210> 1
<211> 116
<212> DNA
<213> Artificial Sequence

<220>
<223> Description of Artificial Sequence: Polynucleotide
      1

<400> 1
cgttgcatat gtggaagaag atcatattgc acatgaatat gcacagagtg ttccttatgg 60
catttctcaa attaaagcgc cggctcttca ctctcaaggc tacacaggct ctaacg     116


<210> 2
<211> 116
<212> DNA
<213> Artificial Sequence

<220>
<223> Description of Artificial Sequence: Polynucleotide
      2

<400> 2
cgttgcatat gtggaagaag atcatattgc acatgaatat gcacagagtg ctccttatgg 60
catttctcaa attaaagcgc cggctcttca ctctcaaggc tacacaggct ctaacg     116


<210> 3
<211> 419
<212> DNA
<213> Artificial Sequence

<220>
<223> Description of Artificial Sequence: Polynucleotide
      3

<400> 3
cgttgcatat gtggaagaag atcatattgc acatgaatat gcacagagtg ttccttatgg 60
catttctcaa attaaagcgc cggctcttca ctctcaaggc tacacaggct ctaacgtaaa 120
agtagctgtt atcgacagcg gaattgactc ttctcatcct gacttaaacg tcagaggcgg 180
agcaagcttc gtaccttctg aaacaaaccc ataccaggac ggcagttctc acggtacgca 240
tgtagccggt acgattgccg ctcttaataa ctcaatcggt gttctgggcg tagcgccaag 300
cgcatcatta tatgcagtaa aagtgcttga ttcaacagga agcggccaat atagctggat 360
tattaacggc attgagtggg ccatttccaa caatatggat gttatcaaca tgagccttg  419

<210> 4
<211> 419
<212> DNA
<213> Artificial Sequence

<220>
<223> Description of Artificial Sequence: Polynucleotide
      4

<400> 4
cgttgcatat gtggaagaag atcatattgc acatgaatat gcacagagtg ctccttatgg 60
catttctcaa attaaagcgc cggctcttca ctctcaaggc tacacaggct ctaacgtaaa 120
agtagctgtt atcgacagcg gaattgactc ttctcatcct gacttaaacg tcagaggcgg 180
agcaagcttc gtaccttctg taacaaaccc ataccaggac agcagttctc acggtacgca 240
tgtagccggt acgattgtcg ctcttaataa ctcaatcggt gttctgggcg tagcgccaag 300
cgcatcatta tatgcagtaa aagtgcttga ttcaacagga agcggcgtat atagctggat 360
tattaacggc attgagtggg ccatttccaa caatatggat gttatcaaca tgagccttg  419